# EUROPEAN PATENT APPLICATION

(11) **EP 4 066 817 A1**
(43) Date of publication of application: **05.10.2022**
(21) Application number: 21166452.9
(22) Date of filing: 31.03.2021
(51) Int. Cl.: A61K 9/00, A61K 31/5575, A61K 47/10, A61K 47/44

(54) **STABLE OPHTHALMIC COMPOSITION COMPRISING LATANOPROST**

(71) Applicant: actrevo GmbH, 20457 Hamburg (DE); OPTUS Pharmaceutical Co., Ltd., Heungdeok-gu, Cheongju-si Chungcheongbuk-do 28158 (KR)
(72) Inventor: FLEMMING, Jens, 22143 Hamburg (DE); PETERS, Harm, 22869 Schenefeld (DE); CHOI, Sunghak, Sungnam-si, Kyunggi-do (KR); LEE, Kangok, Suwon-si, Kyunggi-do (KR); KO, Jinsook, Yongin-si, Kyunggi-do (KR); PARK, Youngwoon, Cheongju-si, Chungcheongbuk-do (KR)
(74) Representative: Dr. Klemens Schubert Patentanwalt

(57) **Abstract**

The present invention refers to an aqueous ophthalmic composition comprising latanoprost in an amount of 0.003 weight-% to 0.007 weight-%, macrogolglycerol hydroxystearate 40 in an amount of 0.3 weight-% to 0.7 weight-%, and propylene glycol in an amount of 0.05 weight-% to 0.09 weight-%, wherein the composition is free of preservatives and stabilizing agents. Furthermore, the present invention refers to the use in the treatment of ocular hypertension and/or glaucoma.

## Description

The present invention relates to a stable aqueous ophthalmic latanoprost composition that is free of any stabilizing agents and preservatives. In addition, a latanoprost composition is provided with at least one further active ingredient, for example selected from timolol and brinzolamide.

The present invention discloses aqueous ophthalmic latanoprost compositions. Latanoprost, isopropyl-(Z)-7-[(1R,2R,3R,5S)-3,5-dihydroxy-2-[(3R)-3-hydroxy-5-phenylpentyl]cyclopentyl]-5-heptenoate, CAS number 130209-82-4, is a prostaglandin F2α analogue and is known for its use in ophthalmic compositions. Especially, latanoprost is proposed as active ingredient in ophthalmic compositions in the treatment of glaucoma and ocular hypertension. As further active ingredients in these compositions brinzolamide and timolol, for example in the form of timolol maleate, are proposed.
Several of these formulations are already commercially available and for example sold under the trademarks Xalatan^{®}, Monoprost^{®}, Xaloptic^{®} and Fixaprost^{®}.

In general, ophthalmic compositions need to be stable and the active ingredient needs to be solubilized to be delivered to the place of action. To this end in many cases high amounts of surfactant are added to the solution, what can lead to side effects like irritations of the eye.

With ophthalmic compositions it is always a challenge to provide a composition that is stable and furthermore does not lead to side effects like irritations of the eye of a patient. Attempts have been made to stabilize latanoprost eyedrops by use of benzalkonium chloride (Xaloptic^{®}) or by adapting the pH value of the ophthalmic solution to 5.0 to 6.25. The respective product was sold under the trademark "Xalatan^{®} pH 6". In both cases side effects like irritations of the eye were reported.

But even if these side effects are tolerated, for example for Xalatan^{®}, the guidelines of "The International Council for Harmonisation of Technical Requirements for Pharmaceuticals for Human Use" (ICH) recommend not to store these compositions above 25 °C. This recommendation is the result of stability testings of the respective compositions. With increasing temperatures in many areas in the world, this recommendation results in the necessity to store the compositions in a refrigerator, if a refrigerator is available at all.

It is therefore an object of the present invention to overcome the drawbacks of the state of the art and to provide an aqueous ophthalmic latanoprost composition that is free of preservatives and stabilizing agents and furthermore shows surprising stability without stabilizing agent.

The object of the present invention is to provide an aqueous ophthalmic composition that comprises no preservatives and no stabilizing agents and shows a high stability even at elevated temperatures and is well tolerated.

The object of the present invention is solved by providing an aqueous ophthalmic composition according to claim 1 and the preferred embodiments according to the dependant claims.

Thus, the object of the invention is solved by providing an aqueous ophthalmic composition comprising latanoprost in an amount of 0.003 weight-% to 0.007 weight-%, macrogolglycerol hydroxystearate 40 in an amount of 0.3 weight-% to 0.7 weight-%, and propylene glycol in an amount of 0.05 weight-% to 0.09 weight-%, wherein the composition is free of preservatives and stabilizing agents.
Macrogolglycerol hydroxystearate 40 is also known as Kolliphor^{®} RH 40 and Polyoxyl 40 hydrogenated castor oil.

The aqueous ophthalmic composition according to the invention is preferred wherein macrogolglycerol hydroxystearate 40 is entirely or substantially entirely the only surfactant in the composition and/or propylene glycol is entirely or substantially entirely the only non-ionic isotonic agent in the composition.

Furthermore preferred is the aqueous ophthalmic composition according to the invention wherein the composition comprises latanoprost in an amount of at least 0.004 weight-%, and/or macrogolglycerol hydroxystearate 40 in an amount of at least 0.4 weight-% and/or propylene glycol in an amount of at least 0.06 weight-%.

Additionally preferred is the aqueous ophthalmic composition according to the invention wherein the composition comprises latanoprost in an amount of at least 0.005 weight-%, and/or macrogolglycerol hydroxystearate 40 in an amount of at least 0.5 weight-% and/or propylene glycol in an amount of at least 0.07 weight-%.

Especially preferred is the aqueous ophthalmic composition according to the invention wherein the composition comprises at least one further active ingredient.
Additionally preferred, is the aqueous ophthalmic composition according to the invention wherein the at least one further active ingredient is selected from timolol and brinzolamide and the pharmaceutically acceptable salts thereof.

In a furthermore preferred embodiment of the aqueous ophthalmic composition according to the invention the at least one further active ingredient is timolol or a pharmaceutical acceptable salt thereof, in an amount of 0.4 to 0.6 weight-% based on the free base timolol.

Additionally preferred, is the aqueous ophthalmic composition according to the invention wherein the at least one further active ingredient is timolol or a pharmaceutical acceptable salt thereof, in an amount of at least 0.5 weight-% based on the free base timolol.

In an especially preferred embodiment of the invention the aqueous ophthalmic composition comprises at least one ionic isotonic agent selected from the group comprising sodium chloride, potassium chloride, magnesium chloride, and calcium chloride.

Furthermore preferred is the aqueous ophthalmic composition according to the invention wherein the composition comprises a buffer system.
In a preferred embodiment according to the invention the aqueous ophthalmic composition comprises a buffer system that is a phosphate buffer system.

Especially preferred, is the aqueous ophthalmic composition according to the invention wherein the pH value of the composition is between 6.4 to 7.0, preferably between 6.5 to 6.9 and especially preferred between 6.6 to 6.8.

In a preferred embodiment according to the invention the aqueous ophthalmic composition is provided as single dose.

Especially preferred is the use of the aqueous ophthalmic composition according to the invention in the treatment of ocular hypertension and/or glaucoma.

Furthermore preferred, is the use of the aqueous ophthalmic composition according to the invention for the treatment of ocular hypertension and/or glaucoma.

Preferably, the aqueous ophthalmic composition according to the invention is provided as eyedrop solution.

### Brief description of the drawings

Fig. 1 shows the results of viability tests of the composition according to the invention, compared to Xalacom^{®} and Xalatan^{®}.
Fig. 2 shows the results of viability tests of the composition according to the invention, compared to Monoprost^{®} and Fixaprost^{®}.

Surprisingly, it was found that the composition according to the invention shows excellent stability even when stored at 40 °C. The results of stability testing show that the composition according to the invention is stable for at least 6 months at 40 °C.

And furthermore, cell viability tests show that in addition the composition according to the invention shows a high tolerability. This means, that different from approaches in the state of the art, the improved stability is not accompanied by side effects or incompatibilities.

The aqueous ophthalmic composition according to the invention comprises latanoprost, macrogolglycerol hydroxystearate 40 (Kolliphor^{®} RH 40), propylene glycol, a buffer system and a ionic isotonic agent, for example sodium chloride, and is adjusted to pH 6.4 to 7.0 by means of sodium hydroxide or hydrochloric acid. No further additives are necessary to accomplish the aim of the invention. And most important, the composition is free of preservatives and stabilizing agents.

Thus, beside the at least one active ingredient latanoprost, the composition according to the invention comprises macrogolglycerol hydroxystearate 40 as surfactant and propylene glycol as solvent and tonicity agent to adjust the osmolality of the composition.

In a preferred embodiment of the aqueous ophthalmic composition according to the invention the buffer system is a phosphate buffer that is prepared from sodium dihydrogen phosphate monohydrate and anhydrous disodium phosphate.

In a preferred embodiment of the aqueous ophthalmic composition according to the invention at least one further active ingredient is included, for example timolol or brinzolamide, or the physiologically acceptable salts of these active ingredients.

Stability tests were performed with a preferred embodiment according to the invention as will be described below.

### Stability data

Using blow-fill-seal technology, the preferred embodiment (0.05 mg/mL latanoprost solution as disclosed in Table 1) is filled into strips of LDPE single-dose container. The single dose containers are labelled. 2 strips of 5 filled ampoules are packed into an aluminium foil pouch.
Stability tests were performed on three batches (19001,19002,19003) of the preferred embodiment under accelerated conditions. The composition of the tested composition is shown in Table 1. Table 2 shows the stability testing under accelerated conditions, and Table 3 shows the results of stability testing under long term conditions.

A single-dose container of the drug product contains not less than 0.2 mL of Latanoprost 0.05 mg/mL eye drops, solution. The drug product is a clear, colourless solution, sterile and practically free from particles. The nominal pH of solution is 6.7 and the osmolality of the solution is 250 to 320 mOsmol/kg.

**Table 1: preferred embodiment - 1 ml of Latanoprost 0.05 mg/mL eye drops, solution in single-dose container, contains:**

| **Name of ingredient** | **Quantity per mL** | **Function** | **Reference to standards** |
|---|---|---|---|
| **Active substance** | | | |
| Latanoprost¹ | 0.05 mg | Active substance | USP² |

| **Excipient(s)** | | | |
|---|---|---|---|
| Sodium chloride | 4.10 mg | Tonicity agent | Ph. Eur.² |
| Sodium dihydrogen phosphate monohydrate | 4.60 mg | Buffer salt | USP² |
| Disodium phosphate anhydrous | 4.74 mg | Buffer salt | Ph. Eur.² |
| Macrogolglycerol hydroxystearate 40 | 5.00 mg | Surfactant | Ph. Eur.² |
| Propylene glycol | 0.70 mg | Solvent, tonicity agent | Ph. Eur.² |
| Sodium hydroxide / Hydrochloric acid ³ | q.s. to pH 6.7 | pH-adjustment | Ph. Eur.² |
| Nitroqen ⁴ | - | Processinq aid | Ph. Eur.² |
| Water for injections ⁵ | add to 1 mL | Vehicle | Ph. Eur.² |
| Total weight | | 1.010 g | |

| | | | |
|---|---|---|---|
| ¹ The quantity of active ingredient is adjusted as per its assay and water content / residual solvents. ² Current edition. ³ Sodium hydroxide and/or hydrochloric acid is used for the adjustment of pH, as required, the excipient is added for example, as a 1 N solution to obtain the specified pH value. The term q.s. means *quantum satis.* ⁴ Not found in finished product. ⁵ Add to 1 mL corresponds to a theoretical amount of about 990.81 mg water for injections. | | | |

**Table 2: Stability testing results - accelerated conditions**

| **Accelerated conditions, 40°C ± 2°C / NMT 25% RH** | | | | |
|---|---|---|---|---|
| **Parameter / Specification limit** | **Batch No.** | **Test time points (months)** | | |
| | | **0** | **3** | **6** |
| **Appearance of container** | 19001 | complies | complies | complies |
| Transparent LDPE single dose container | 19002 | complies | complies | complies |
| | 19003 | complies | complies | complies |
| **Appearance of solution** | 19001 | complies | complies | complies |
| Clear solution; practically free from particles | 19002 | complies | complies | complies |
| | 19003 | complies | complies | complies |
| **Colour** | 19001 | complies | complies | complies |
| Colourless solution | 19002 | complies | complies | complies |
| | 19003 | complies | complies | complies |
| **pH** | 19001 | 6.8 | 6.8 | 6.7 |
| 6.7 ± 0.3 (6.4 to 7.0) | 19002 | 6.8 | 6.8 | 6.7 |
| | 19003 | 6.7 | 6.7 | 6.7 |
| **Absolute density** | 19001 | 1.009 | 1.010 | 1.010 |
| 1.010 ± 0.010 g/mL (1.000 to 1.020 g/mL) | 19002 | 1.010 | 1.004 | 1.009 |
| | 19003 | 1.009 | 1.007 | 1.010 |
| **Osmolality** | 19001 | 278 | 280 | 279 |
| 250 to 320 mOsmol/kg | 19002 | 277 | 284 | 280 |
| | 19003 | 278 | 283 | 290 |
| **Extractable volume [mL]** | 19001 | 0.2249 | NA | 0.2408 |
| NLT 0.2 mL (equal to 0.2 g) | 19002 | 0.2058 | NA | 0.2228 |
| | 19003 | 0.2337 | NA | 0.2113 |
| **Loss of Water [%]** | 19001 | NA | 0.14% | 0.17% |
| NMT 5 % from To | 19002 | NA | 0.15% | 0.28% |
| | 19003 | NA | 0.29% | 0.46% |
| **Mean droplet size [mL]** | 19001 | 0.030 | 0.030 | 0.029 |
| 0.03 mL ± 10 % (0.027 to 0.033 mL) | 19002 | 0.030 | 0.028 | 0.030 |
| | 19003 | 0.030 | 0.029 | 0.030 |
| **Closure integrity** | 19001 | complies | NA | complies |
| No leaking / no penetration of the color | 19002 | complies | NA | complies |
| | 19003 | complies | NA | complies |
| **Identification latanoprost** | 19001 | complies | complies | complies |
| Retention time of the major peak in the chromatogram of the sample solution corresponds to that of the standard solution obtained in the test of assay (± 0.5 minute) | 19002 | complies | complies | complies |
| | 19003 | complies | complies | complies |
| **Assay latanoprost [%]** | 19001 | 98.2% | 100.0% | 98.3% |
| 0.0475 to 0.0525 mg/mL corresponding to 95.0 to 105.0% of declaration | 19002 | 97.7% | 99.9% | 98.0% |
| | 19003 | 97.5% | 100.7% | 98.8% |
| **Impurities [%]** | 19001 | ND | 0.7% | 0.8% |
| 5,6-trans Latanoprost: NMT 4.0 % | 19002 | 2.8% | 3.7% | 3.8% |
| | 19003 | 2.8% | 3.7% | 3.6% |
| 15-S Latanoprost: NMT 1.0 % | 19001 | ND | 0.4% | ND |
| | 19002 | ND | ND | ND |
| | 19003 | ND | ND | ND |
| Latanoprost acid: NMT 1.0 % | 19001 | ND | 0.5% | 0.4% |
| | 19002 | ND | 0.4% | 0.4% |
| | 19003 | ND | 0.4% | 0.4% |
| Any other individual unspecified impurity: NMT 1.0 % | 19001 | complies | complies | complies |
| | 19002 | complies | complies | complies |
| | 19003 | complies | complies | complies |
| Total impurities (excluding 5,6-trans and 15-S Latanoprost): NMT 3 % | 19001 | complies | complies | complies |
| | 19002 | complies | complies | complies |
| | 19003 | complies | complies | complies |
| **Sterility** | 19001 | sterile | NA | sterile |
| Sterile | 19002 | sterile | NA | sterile |
| | 19003 | sterile | NA | sterile |

| | | | | |
|---|---|---|---|---|
| NLT-not less than; NMT-not more than; NT-not tested; NA-not applicable; ND-not detected. | | | | |

**Table 3: Stability testing results - long term conditions (II)**

| **Long term conditions (II), 25 ± 2°C / 40 ± 5% RH** | | | | | |
|---|---|---|---|---|---|
| **Parameter/ Specification limit** | **Batch No.** | **Test time points (months)** | | | |
| | | **0** | **3** | **6** | **9** |
| **Appearance of container** | 19001 | complies | complies | complies | complies |
| | 19002 | complies | complies | complies | complies |
| Transparent LDPE single dose container | 19003 | complies | complies | complies | complies |
| **Appearance of solution** | 19001 | complies | complies | complies | complies |
| | 19002 | complies | complies | complies | complies |
| Clear solution; practically free from particles | 19003 | complies | complies | complies | complies |
| **Colour** | 19001 | complies | complies | complies | complies |
| Colourless solution | 19002 | complies | complies | complies | complies |
| | 19003 | complies | complies | complies | complies |
| **pH** | 19001 | 6.8 | 6.8 | 6.7 | 6.7 |
| 6.7 ± 0.3 (6.4 to 7.0) | 19002 | 6.8 | 6.8 | 6.7 | 6.8 |
| | 19003 | 6.7 | 6.8 | 6.8 | 6.7 |
| **Absolute density** | 19001 | 1.009 | 1.009 | 1.009 | 1.009 |
| 1.010 ± 0.010 g/mL (1.000 to 1.020 g/mL) | 19002 | 1.010 | 1.005 | 1.009 | 1.009 |
| | 19003 | 1.009 | 1.009 | 1.009 | 1.009 |
| **Osmolality** | 19001 | 278 | 281 | 278 | 281 |
| 250 to 320 mOsmol/kg | 19002 | 277 | 282 | 276 | 283 |
| | 19003 | 278 | 281 | 278 | 279 |
| **Extractable volume [mL]** | 19001 | 0.2249 | NA | 0.2259 | NA |
| | 19002 | 0.2058 | NA | 0.2318 | NA |
| NLT 0.2 mL (equal to 0.2 g) | 19003 | 0.2337 | NA | 0.2373 | NA |
| **Loss of Water [%]** NMT 5 % from To | 19001 | NA | 0.03 | 0.02 | 0.03 |
| | 19002 | NA | 0.01 | 0.07 | 0.05 |
| | 19003 | NA | 0.03 | 0.11 | 0.08 |
| **Mean droplet size [mL]** 0.03 mL ± 10 % (0.027 to 0.033 mL) | 19001 | 0.030 | 0.030 | 0.030 | 0.031 |
| | 19002 | 0.030 | 0.029 | 0.030 | 0.030 |
| | 19003 | 0.030 | 0.029 | 0.030 | 0.030 |
| **Closure integrity** No leaking / no penetration of the color | 19001 | complies | NA | complies | NA |
| | 19002 | complies | NA | complies | NA |
| | 19003 | complies | NA | complies | NA |
| **Identification latanoprost** | 19001 | complies | complies | complies | complies |
| Retention time of the major peak in the chromatogram of the sample solution corresponds to that of the standard solution obtained in the test of assay (± 0.5 minute) | 19002 | complies | complies | complies | complies |
| | 19003 | complies | complies | complies | complies |
| **Assay latanoprost [%]** 0.0475 to 0.0525 mg/mL corresponding to 95.0 to 105.0% of declaration | 19001 | 98.2 | 100.2 | 99.8 | 101.2 |
| | 19002 | 97.7 | 98.9 | 97.6 | 100.4 |
| | 19003 | 97.5 | 99.8 | 99.3 | 101.0 |
| **Impurities [%]** 5,6-trans Latanoprost: NMT 4.0 % | 19001 | ND | 0.2 | 0.4 | 0.7 |
| | 19002 | 2.8 | 3.1 | 3.6 | 3.5 |
| | 19003 | 2.8 | 3.1 | 3.4 | 3.6 |
| 15-S Latanoprost: NMT 1.0 % | 19001 | ND | 0.3 | ND | ND |
| | 19002 | ND | ND | 0.8 | ND |
| | 19003 | ND | ND | 0.3 | ND |
| Latanoprost acid: NMT 1.0 % | 19001 | ND | 0.1 | 0.2 | 0.2 |
| | 19002 | ND | 0.1 | 0.2 | 0.2 |
| | 19003 | ND | 0.1 | 0.2 | 0.2 |
| 15-keto-Latanoprost: NMT 1.0 % | 19001 | NT | NT | NT | NT |
| | 19002 | NT | NT | NT | NT |
| | 19003 | NT | NT | NT | NT |
| Any other individual unspecified impurity: NMT 1.0 % | 19001 | complies | complies | complies | complies |
| | 19002 | complies | complies | complies | complies |
| | 19003 | complies | complies | complies | complies |
| Total impurities (excluding 5,6-trans and 15-S Latanoprost): NMT 3 % | 19001 | complies | complies | complies | complies |
| | 19002 | complies | complies | complies | complies |
| | 19003 | complies | complies | complies | complies |
| **Sterility** | 19001 | sterile | NA | sterile | NA |
| Sterile | 19002 | sterile | NA | sterile | NA |
| | 19003 | sterile | NA | sterile | NA |

| | | | | | |
|---|---|---|---|---|---|
| Note: The specified impurity 15-keto-Latanoprost was included in the drug product specification; before including these data 15-keto-Latanoprost was evaluated under "any unspecified impurity" NLT-not less than; NMT-not more than; NT-not tested; NA-not applicable; ND-not detected. | | | | | |

After the outstanding stability of the composition according to the invention was revealed, the inventors investigated whether the presence of propylene glycol in the composition according to the invention has an effect on the stability of the composition.

To this end, the stability of two composition was compared. Composition RD20-131 is a composition according to the invention and therefore comprising propylene glycol. The composition is shown in Table 4.
Composition RD20-132, shown in Table 5, differs from RD20-131 only in that RD20-132 does not comprise propylene glycol.

**Table 4: Batch No. RD20-131**

| 1 mL solution of Batch No. RD20-131 contains: | |
|---|---|
| 0.05 mg | latanoprost, |
| 4.10 mg | sodium chloride |
| 4.60 mg | sodium dihydrogen phosphate monohydrate |
| 4.47 mg | disodium phosphate anhydrous |
| 5.00 mg | macrogolglycerol hydroxystearate 40 |
| 0.70 mg | propylene glycol |
| sodium hydroxide or hydrochloric acid for pH adjustment | |
| and water ad 1.0 mL | |

RD20-132 has the same composition, but without propylene glycol.

**Table 5: Batch No. RD20-132**

| 1mL solution of Batch No. RD20-132 contains: | |
|---|---|
| 0.05 mg | latanoprost, |
| 4.10 mg | sodium chloride |
| 4.60 mg | sodium dihydrogen phosphate monohydrate |
| 4.47 mg | disodium phosphate anhydrous |
| 5.00 mg | macrogolglycerol hydroxystearate 40 |
| sodium hydroxide or hydrochloric acid for pH adjustment | |
| and water ad 1.0 mL | |

Both compositions (RD20-131 and RD20-132) were stored at 2 °C to 8 °C (as control) and for 5 days at 60 °C.
In order to determine the stability of the compositions, the appearance of the solutions, the pH value, the osmolality, the assay and the impurities were determined.

As the results summarized in Table 6 show, the presence of propylene glycol does not influence the stability of the solution. Both compositions, namely RD20-131 and RD20-132 show the same stability.

**Table 6: Investigation of possible influence of propylene glycol on stability**

| **Batch No.** | **RD20-131** | **RD20-131** | **RD20-132** | **RD20-132** |
|---|---|---|---|---|
| Composition | with propylene glycol | | without propylene glycol | |
| Storage condition | Control (2 - 8 °C) | 5 days at 60 °C | Control (2 - 8 °C) | 5 days at 60 °C |
| Appearance | Clear, colourless solution | Clear, colourless solution | Clear, colourless solution | Clear, colourless solution |
| pH | 6.79 | 6.77 | 6.70 | 6.80 |
| Osmolality [mOs-mol/kg]: | 282 | 281 | 272 | 271 |
| Assay [%]: | 98.2 | 97.6 | 97.4 | 96.6 |
| Impurities [%]: | 2.82 | 2.85 | 2.79 | 2.83 |

This means that propylene glycol does not have a stabilizing effect on the composition according to the invention and therefore cannot be regarded to be a stabilizing agent.
Much rather, propylene glycol seems to increase the solubilization of the active ingredient and aids in adjusting the osmolality.

As already mentioned, high stability of ophthalmic compositions is often accompanied by deleterious side effects to the user. The reasons for side effects are in most cases the preservatives and stabilizing agents used, or further stabilizing parameters of the solutions like the pH value.

### Safety and tolerability

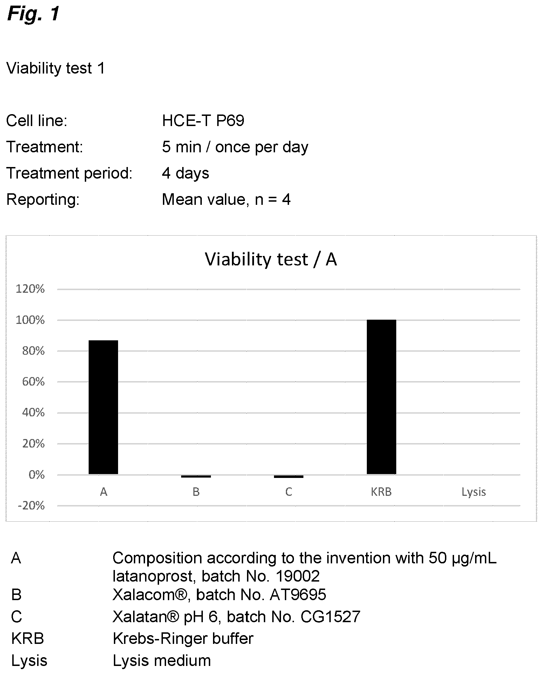

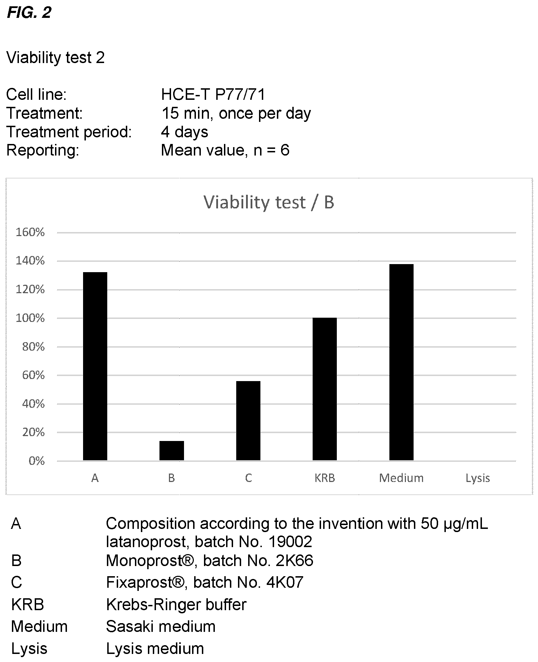

In order to investigate the safety and tolerability of the compositions according to the invention cell viability tests were performed according to the cell proliferation assay. To this end the influence of the latanoprost composition on cell proliferation and cell viability were studied using HCE-T cells. The results of the investigation were compared to the results of in parallel performed tests on commercially available latanoprost compositions. Figures 1 and 2 show the graphical representations of the results of the test models. The test and the results are detailed in the following.

The tests show the high acceptance of the composition according to the invention, and especially a very high acceptance compared to Xalacom^{®}, Xalatan^{®}, Monoprost^{®}, and Fixaprost^{®}.

### Test model cell proliferation assay (cell viability test)

Test model: HCE-T cells, wherein HCE-T stands for "immortalized human corneal epithelial cell line".
Testing procedure: The general procedure followed the approach described by Huhtala et al. in J. Ocul. Pharmacol. Ther. 2003, 19(1), 11-21. Immortalized HCE-T cells were cultivated following internal procedures. The cells were exposed to formulations for 15 min once per day over 4 days. Thereafter, the viability of cells was determined by MTS assay (see Malich et al. in Toxicology 1997, 124(3), 179-192). For both negative and positive control of test model the cells were treated with Krebs-Ringer and lysis buffer medium as well.

The viability tests were performed in comparison with commercially available latanoprost compositions. In the following the composition of these commercially available is indicated based on the published information.

### Xalatan^{®} pH 6

Xalatan^{®} 50 micrograms/mL latanoprost eye drops solution comprise in 1 mL 0.05 mg Latanoprost, 0.20 mg benzalkonium chloride, sodium chloride, 7.70 mg sodium dihydrogen phosphate monohydrate, 1.55 mg anhydrous disodium phosphate, and water for injections

There are no further data publicly available.

### Xalacom^{®}

Xalacom eye drops, solution in multi-dose container comprise in 1 mL 0.05 mg latanoprost, 6.80 mg timolol maleate what corresponds to 5.00 mg timolol free base, sodium dihydrogen phosphate monohydrate, anhydrous disodium phosphate, sodium chloride, benzalkonium chloride and water for injections.

### Monoprost^{®}

Monoprost^{®} 50 micrograms/mL latanoprost eye drops solution comprise in 1 mL 0,05 mg latanoprost, 50 mg macrogolglycerol hydroxystearate 40, sorbitol, carbomer 974 P, Macrogol 4000, disodium edetate, sodium hydroxide and water for injections.

This means that the composition comprises a comparably high amount of surfactant, that can lead to a comparably reduced tolerability of the composition.

### Fixaprost^{®}

Fixaprost^{®} comprises in 1 mL eye drops solution 0.05 mg latanoprost, 5 mg timolol (in form of timolol maleate), 50 mg macrogolglycerol hydroxystearate, sorbitol, macrogol 4000, Carbomer 974P, sodium edetate, sodium hydroxide and water for injection.

As can be derived from the results of the viability test the composition according to the invention shows a higher degree of tolerability compared to the parallel tested products. The compositions Xalatan^{®} and Xalacom^{®} both comprise benzalkonium chloride that is known to show toxic effect on HCE-T cells. This effect can be seen in the viability data in Fig. 1.

Furthermore, the data provided in Fig. 2 show that Monoprost^{®} and Fixaprost^{®} are less tolerable than the composition according to the invention. Both compositions comprise a much higher amount of surfactant, namely 50 mg/mL compared to 5 mg/mL in the composition according to the invention.

Thus, it was shown that the composition according to the invention is more tolerable and less deleterious to the eye than the compared compositions.

### Combination with timolol

In a further preferred embodiment, the composition according to the invention comprises timolol, preferably in the form of timolol maleate. Timolol is a non-selective β-blocker and is combined with latanoprost in the treatment of ocular hypertension.

Timolol is added to the above referenced preferred embodiments of the latanoprost composition in an amount of 0.4 to 0.6 weight-%, most preferred 0.5 weight-%, based on the free base of timolol.

The most preferred composition according to the invention comprising timolol and latanoprost contains the following components in 1 mL as shown in Table 7.

**Table 7: Composition according to the invention with timolol**

| | |
|---|---|
| 0.05 mg | latanoprost, |
| 6.80 mg | timolol maleate (corresponds to 5.00 mg timolol free base), |
| 4.60 mg | sodium dihydrogen phosphate monohydrate |
| 4.74 mg | anhydrous disodium phosphate |
| 2.95 mg | sodium chloride |
| 5.00 mg | macrogolglycerol hydroxystearate 40 |
| 0.70 mg | propylene glycol |
| | sodium hydroxide / hydrochloric acid for pH adjustment and |
| | water for injections. |
| The pH value of the composition is pH 6.7 ± 0.1. | |

The composition according to the invention wherein the at least one further active ingredient is timolol, shows the same positive characteristics in respect of storage stability and tolerance as the composition according to the invention without the second active ingredient.

The reason is that according to the invention no preservative and no stabilizing agent is included in the compositions and furthermore, the compositions according to the invention have a comparably low content of surfactant.

The aqueous ophthalmic composition according to the invention can be provided in any ophthalmic acceptable form like an solution, emulsion, suspension, gel and ointment.

## Claims

1. An aqueous ophthalmic composition comprising latanoprost in an amount of 0.003 weight-% to 0.007 weight-%, macrogolglycerol hydroxystearate 40 in an amount of 0.3 weight-% to 0.7 weight-%, and propylene glycol in an amount of 0.05 weight-% to 0.09 weight-%, wherein the composition is free of preservatives and stabilizing agents.

2. The aqueous ophthalmic composition according to claim 1, **characterized in that** macrogolglycerol hydroxystearate 40 is entirely or substantially entirely the only surfactant in the composition and/or propylene glycol is entirely or substantially entirely the only non-ionic isotonic agent in the composition.

3. The aqueous ophthalmic composition according to any of the preceding claims, **characterized in that** the composition comprises latanoprost in an amount of at least 0.004 weight-%, and/or macrogolglycerol hydroxystearate in an amount of at least 0.4 weight-% and/or propylene glycol in an amount of at least 0.06 weight-%.

4. The aqueous ophthalmic composition according to any of the preceding claims, **characterized in that** the composition comprises latanoprost in an amount of at least 0.005 weight-%, and/or macrogolglycerol hydroxystearate 40 in an amount of at least 0.5 weight-% and/or propylene glycol in an amount of at least 0.07 weight-%.

5. The aqueous ophthalmic composition according to any of the preceding claims, **characterized in that** the composition comprises at least one further active ingredient.

6. The aqueous ophthalmic composition according to claim 5, **characterized in that** the at least one further active ingredient is selected from timolol and brinzolamide and the pharmaceutically acceptable salts thereof.

7. The aqueous ophthalmic composition according to claim 6, **characterized in that** the at least one further active ingredient is timolol or a pharmaceutical acceptable salt thereof, in an amount of 0.4 to 0.6 weight-% based on the free base timolol.

8. The aqueous ophthalmic composition according to claim 7, **characterized in that** the at least one further active ingredient is timolol or a pharmaceutical acceptable salt thereof, in an amount of at least 0.5 weight-% based on the free base timolol.

9. The aqueous ophthalmic composition according to any of the preceding claims, **characterized in that** the composition comprises at least one ionic isotonic agent selected from the group comprising sodium chloride, potassium chloride, magnesium chloride, and calcium chloride.

10. The aqueous ophthalmic composition according to any of the preceding claims, **characterized in that** the composition comprises a buffer system.

11. The aqueous ophthalmic composition according to claim 10, **characterized in that** the buffer system is a phosphate buffer system.

12. The aqueous ophthalmic composition according to any of the preceding claims, **characterized in that** the pH value of the composition is between 6.4 to 7.0, preferably between 6.5 to 6.9, and especially preferred between 6.6 to 6.8.

13. The aqueous ophthalmic composition according to any of the preceding claims, **characterized in that** the composition is provided as single dose.

14. Use of the aqueous ophthalmic composition according to any of the preceding claims in the treatment of ocular hypertension and/or glaucoma.

15. Use of the aqueous ophthalmic composition according to any of the preceding claims for the treatment of ocular hypertension and/or glaucoma.
